# EUROPEAN PATENT SPECIFICATION

(11) **EP 2 155 852 B1**
(45) Date of publication and mention of the grant of the patent: **09.10.2013**
(21) Application number: 08766811.7
(22) Date of filing: 16.06.2008
(51) Int. Cl.: C12M 1/00, C12M 1/02, B01F 11/00, B01F 15/00

(54) **IMPROVED FLEXIBLE BIOREACTOR**
VERBESSERTER FLEXIBLER BIOREAKTOR
BIORÉACTEUR FLEXIBLE AMÉLIORÉ

(30) Priority: 15.06.2007 EP 07110320
(43) Date of publication of application: 24.02.2010
(73) Proprietor: Cellution Biotech B.V., 9403 AC Assen (NL)
(72) Inventor: OOSTERHUIS, Nicolaas Marius Gerard, NL-9451 KT Rolde (NL); PEETERS, Marc Alfons Eugeen, B-3128 Baal-tremelo (BE); VAN DER HEIDEN, Pieter, NL-4725 SN Wouwse Plantage (NL)
(74) Representative: Hatzmann, Martin
(86) International application number: PCT/NL2008/050388
(87) International publication number: WO 2008/153401

(56) References cited:
- WO-A-03/028869
- WO-A-2006/017951
- US-A1- 2004 027 912

## Description

The invention relates to the field of biotechnology and culturing of cells in a liquid growth medium. Among others, it relates to flexible, single-use bioreactor bags specifically adapted to allow for detachably securing the bioreactor to a bioreactor support structure. It relates in particular to a flexible bioreactor specifically adapted for non-invasive and accurate monitoring of the cellular, e.g. metabolic, activity of cells. It also relates to an apparatus for cultivating cells utilizing the improved bioreactor, and to methods for cultivating cells.

Flexible disposable bioreactors for culturing cells in a liquid medium are known in the art. US 6,190,913 discloses the use of an inflated plastic bag that provides a sterile, disposable cultivation chamber for various types of cells. The bag is placed on a rocking platform to induce a gentle wave-like motion to the liquid contained therein, providing liquid mixing and enhancing oxygen transfer from the headspace gas to the liquid phase where it is essential for cell growth and metabolism. Restraining straps prevent the bag from slipping of the platform. Rocking rate is 1 to 30 rocks per minute.

EP1173542 also relates to a bioreactor bag for culturing cells using wave motion. Therein, the bag is held on opposite sides in a basin by means of a clamping device. The basin is arranged in an oscillating frame, which is capable of oscillating about a horizontal axis of oscillation. A similar construction for retaining a bioreactor on a moving or rocking platform is proposed in W02006/017951.

WO03/028869 discloses a mixing bag or vessel having a receiver for a fluid-agitating element, e.g. a magnetic impeller. The vessel has a rigid portion including a first receiver for receiving and holding the fluid-agitating element at a home location when positioned in the vessel.

One aspect of the present invention relates to methods for securing a flexible bioreactor to its support structure. Disclosed is a disposable bioreactor, e.g. a bioreactor bag, that can be detachably and stably secured to a support structure in a predetermined position, and which requires only minimal handling. A specific aspect relates to a flexible bioreactor bag that can be secured to a rocking platform, ensuring accurate and stable positioning of the bag during use, even if using high agitation speeds e.g. more than 40-50 rpm as disclosed in W02007/00173. WO2005/068059 describes a flexible vessel for bioprocessing comprising a rigid receiver creating a port for receiving a sensor.

Disclosed is a flexible bioreactor intended for culturing cells in a fluid comprising a liquid medium, the bioreactor comprising a flexible wall forming a fluid impermeable bottom wall of the bioreactor when in use, wherein flexible parts of said flexible bottom wall are interrupted by at least one rigid element of said flexible bottom wall, the rigid element allowing for detachably securing the bottom wall of the bioreactor to a support structure for receiving or retaining the bioreactor. The at least one rigid element allows for accurate positioning of the bioreactor in a bioreactor support structure, acting in co-operation with an adaptor element provided at the support structure when the bioreactor is in use. For example, a bioreactor comprising multiple rigid elements in its flexible bottom wall is detachably secured via a corresponding number of adaptor elements on the support structure. In contrast to a fully flexible wall, the locally "rigidified" bottom of the bioreactor can withstand the pressure and handling required to attach and detach the bioreactor safely to a bioreactor housing. Any type of suitable construction pair can be used for the rigid element-adaptor element combination, e.g. a plug-and-socket construction. It may involve a clicking mechanism and/or the use of wedge-shaped constructions to provide a detachable securing system.

In a specific aspect, the rigid element is arranged for cooperation with an adaptor element such that the rigid element and the adaptor element are securable to one another using vacuum pressure, the adaptor element being arranged for being fixedly connected to the support structure. Once a bioreactor of the invention is correctly positioned on the housing, vacuum is applied e.g. through the adaptor element to secure the bioreactor in its position. During operation, for example during rocking, vacuum is maintained. After completion of the culturing process, vacuum is released and the bioreactor can be conveniently removed from the support structure. A positive pressure can be applied to further facilitate release from the adaptor element. This method does not require extensive manual detachment at the bottom of the bioreactor, which may be cumbersome and risky in case the bioreactor is filled with a large volume of (finished) cell culture.

The at least one rigid element can have various shapes and dimensions. They are preferably small enough to allow for a compact volume of the flexible bioreactor when not in use. In general, the total area of the rigid bottom segments does not exceed 10 percent of the total bottom wall of the bioreactor, preferably not more than 5 percent. The cross-section of the rigid element can be circular, rectangular or square, but other shapes are also encompassed. The total number of individual rigid elements can also vary. Also in view of the manufacture process, it is preferred that the bottom contains up to 10, preferably up to 8, more preferably up to 6, most preferably up to 4 rigid elements. The relative positioning of the rigid elements can be freely chosen. In view of a stable and balanced securing of a bioreactor, a more or less even distribution can be preferred. The rigid elements can be positioned along an axis of symmetry, preferably a central axis of symmetry. In case the effective volume of a bioreactor can be modulated (see for example W02007/00173 and further herein below) it is practical to position the rigid elements in the section of the bottom wall that, when in use, forms the bottom of the bioreactor in its minimal volume configuration.

The skilled person will understand that the total number, shape and dimension of the rigid elements, as well as their spatial orientation, are adjusted to the total number, shape, dimension and spatial orientation of the corresponding adaptors on the housing.

The rigid element can be connected fluid tightly to the flexible bottom wall of the bioreactor by various means. Depending on the respective materials of the flexible and rigid bottom sections, the sections may be sealed together to form a fluid tight connection. For example, a flange piece comprising the rigid element can be tightly sealed to the flexible wall parts by heating.

Alternatively, for instance if the materials can not be sealed together, the rigid element comprises a coupling element that is sealingly connected to the flexible parts of the bottom wall to form a fluid tight connection therewith, while the rigid element further comprises an insert element that is sealingly connected to the coupling element to form a fluid tight connection therewith. The coupling element is for instance a hose barb holding the insert element by clamping, the insert element for that purpose preferably being slightly tapered or otherwise wedge-shaped.

The flexible walls are generally made of a transparent or translucent flexible plastic to allow light into the bioreactor and to permit visual inspection of the bioproduction/bioprocessing if desired. Suitable flexible plastics are PVC, polyethylene, Polypropylene, Polyurethane, Hypalon®, HDPE, EPDM, XR-5™, Coolguard™, Teflon®. The type and thickness of plastic will depend upon several variables, including the size of the support structure.

Suitable materials for the rigid element comprise polyethylene, polycarbonate, and similar polymer materials. As will be elaborated on further below, in one embodiment the rigid element is made of a transparent, non-fluorescent material.

The term "rigid" as used herein refers to any inflexible or stiff material that is incapable of or resistant to excessive bending or deformation. A rigid element is made of a material that is less flexible than the material of the flexible parts of the wall forming the bottom of the bioreactor when in use. However, the material of the rigid element and that of the flexible wall can be the same or different in terms of their chemical composition. It is preferred that both materials can withstand the type of sterilization process that will be utilized to sterilize the bioreactor.

In one aspect, the flexible parts of the bottom wall and the rigid element are made from the same material, e.g. the bottom wall of a flexible polyethylene and the at least one rigid element being made of hardened polyethylene. As indicated above, the materials of the rigid element and flexible parts of the bottom wall can be chosen such that they allow for sealing the rigid and flexible materials together, thereby forming a fluid tight connection. In another aspect, the material of the rigid element and that of the flexible parts of the bottom wall are different in terms of their chemical composition. The flexible parts of the bottom wall can be formed by flexible polyethylene sheets to which a polyethylene coupling element of the rigid element is sealed. In that embodiment, the rigid element comprises an insert element being made of a transparent material, such as polycarbonate. The transparent insert element can be clamped in the coupling element.

The invention also relates to a method for cultivating cells in a fluid comprising a liquid medium using a flexible bioreactor, preferably using wave motion, comprising detachably securing a bioreactor to a bioreactor support structure by means present in the bottom wall of the bioreactor that act together with means present on the support structure. Preferably, said means comprises one or more rigid elements and adaptor elements according to the invention.

It is an aim of the present invention to provide a flexible bioreactor that is specifically adapted for non-invasive and accurate monitoring, preferably on-line monitoring, of the cellular, e.g. metabolic, activity of cells. It is a specific aim to provide a bioreactor bag that allows for a reliable and accurate on-line reading, in particular if cells are cultured using wave motion. Flexible bioreactors comprising means for monitoring the metabolic activity of cells are known in the art. For example, W02006/017951 discloses a bag-shaped flexible bioreactor with a housing support arranged on the upper side of the reactor bag. The housing support extends into the bag's interior and comprises a transparent sensor piece and an indicator tile in contact with the contents of the bioreactor and which may be scanned by a fibre optic which may itself be introduced into the housing support and connected to a display device. The housing support is provided with a flange piece tightly sealed to the vessel wall. W02006/017951 indicates that the flange piece may be connected to the sensor piece by means of a tubular connector piece, whereby the length of the connector piece is such that the sensor piece permanently reaches into the reactant

Whereas the arrangement of the sensor and fibre optic proposed in WO2006/017951 may allow for taking a sterile measurement of the metabolic activity of cells, the present inventors experienced that it is very difficult to obtain a reliable and accurate on-line reading, in particular if cells are cultured using wave motion. Culturing cells using wave motion is known in the art, see for example US 6,190,913 and WO2007/00173. It typically comprises the use of a bag-shaped bioreactor secured to a rocking platform that is rocked in a predetermined manner. The rocking motion promotes wave formation in the bag which provides liquid mixing and enhances oxygen transfer from the headspace gas to the liquid phase where it is essential for cell growth and metabolism. However, when using the bioreactor of W02006/017951, a rocking motion often causes the housing support to sway out of the growth medium such that the sensor piece is temporarily in contact with the gaseous phase in the bag's interior, instead of with the reactants. As a result, the signal obtained, e.g. pH or oxygen level, does not accurately reflect the metabolic activity of the cells.

Another drawback of the arrangement set out in W02006/017951 is related to the fact that the fibre optic is part of the housing sticking into the medium and thus also undergoes a wave motion. This motion negatively affects the accuracy of the reading, possible due to a variation in the distance between the tip of the fibre optic and the sensor piece.

The present inventors surprisingly discovered that a rigid element in the bottom of a flexible bioreactor can be advantageously used for monitoring cellular activity. More specifically, the rigid bottom section can not only be used as means for securing the bottom of the bioreactor to a support structure, it can alternatively or additionally serve as carrier for means that allow for non-invasive monitoring of cellular activity, for instance wherein the means comprise a sensor element. Therefore, the invention provides a flexible, bioreactor intended for culturing cells in a liquid medium, the bioreactor comprising a flexible wall forming a fluid impermeable bottom wall of the bioreactor when in use, wherein flexible parts of said flexible bottom wall are interrupted by at least one rigid element of said flexible bottom wall, the rigid element and the flexible parts being connected fluid tightly, the rigid element comprising measuring means allowing for non-invasive (on-line) monitoring of cellular activity in said bioreactor when in use, wherein the rigid element comprises a wall surrounding at least one measuring means, thereby forming a measuring vessel.

As used herein, said "means allowing for non-invasive monitoring" refers to any element, structure or device which is involved or instrumental in the monitoring of cellular activity which avoids the necessity to access the sterile interior of the bioreactor. Thus, it not only encompasses measuring means per se, but also means that enable, or facilitate the actual measuring taking place outside of the bioreactor.

In one embodiment, the measuring means are formed by the rigid element having a surface, preferably a planar surface, of a transparent, non-fluorescent material, said surface being provided with at least one sensor element facing the interior of the bioreactor. Therefore, in one aspect the invention flexible parts of said flexible bottom wall are interrupted by at least one rigid element of said flexible bottom wall, wherein the rigid element has a surface, preferably a planar surface, of a transparent, non-fluorescent material, said surface being provided with at least one sensor element facing the interior of the bioreactor.

In combination with a fiber optic probe positioned below the bottom of the bioreactor, e.g. a probe mounted to the support structure, this arrangement of sensor and probe allows for an accurate and reliable non-invasive monitoring of metabolic activity in the bioreactor. Advantageously, the probe is held in the adaptor element mounted to the support structure and arranged in a manner that allows for detecting the sensor element of the corresponding rigid element. In contrast to the arrangement disclosed in WO2006/017951, the sensor, being part of the bottom, cannot sway out of the cell culture and will be in better contact with the reactants. In addition, the optic fiber probe is not part of the bioreactor and therefore not subjected to the wave motion. The secure and accurate position of the bioreactor comprising the at least one sensor, for instance using vacuum, ensures that the distance between sensor element and optic fiber probe varies only minimally, even at high rocking rates.

It is remarked that a rigid element being provided with at least one measuring means, like a sensor element, is hereinafter referred to as "sensoring rigid element". A rigid element not being provided with a measuring means is hereinafter referred to as "securing rigid element". It is however to be understood that a "sensoring rigid element" can also serve as securing means. Unless indicated otherwise, the term "rigid element" refers to a "sensoring rigid element" as well as to a "securing rigid element".

Sensor elements for use in the present invention are known in the art. It is for example an oxygen sensor or a pH sensor. More preferably, at least an oxygen sensor and a pH sensor are present. The sensor may contain a luminescent or fluorescent dye. Alternatively, it can be a sterilizable electrode. In one embodiment, a rigid element is provided with a conventional pH electrode based on selective diffusion of protons through pH glass, and the determination of potentials between the internal electrolyte and a reference electrode. The reference electrode can be either external (separate sensor) or internal. The electrode is mounted in the rigid insert, e.g. using silicon to achieve a liquid tight connection, such that the pH sensitive tip is in contact with the liquid contents of the bioreactor.

The different sensors are preferably provided on separate rigid elements, preferably on, or through a planar surface of the rigid element, although it is also envisaged that multiple sensor elements are contained in a single rigid element. The sensor signal is conveniently read out from the outside through the transparent, non-fluorescent material of the rigid element, preferably in a PC-controlled manner.

Suitable sensors include planar sensor elements, for instance the sensor spots supplied by PreSens Precision Sensing GmbH, Regensburg; Germany. These cannot be mounted on the bottom of flexible bioreactors known in the art; the flexible, transparent, inert plastic materials used for the bioreactor are not compatible with the conditions required for attaching the sensor element, for instance by gluing. In contrast, a rigid element of the invention can be made of a transparent, non-fluorescent rigid material of choice on which a sensor element can be easily mounted. Polycarbonate is very suitable. In another embodiment, the means allowing for non-invasive monitoring cellular activity comprise the rigid element being interrupted by a membrane allowing the passage of at least one analyte to the exterior of the bioreactor, the analyte being an indicator of cellular activity. Upon passage of the analyte through the membrane by passive diffusion, the analyte can be taken up and transported by a transport medium, preferably a fluid in which the analyte is soluble, to an analyte testing chamber capable of detecting the analyte. This concept *per* se is known in the art, see for example EP1870033. However, the use of a membrane in a rigid insert located at the bottom of a flexible bioreactor has never been disclosed or suggested. Said membrane is for instance a dialysis membrane or filter membrane with a selected pore size. In a specific embodiment, the rigid element is provided with a cellulose acetate membrane. The membrane can be sealed or connected to the rigid element by methods known in the art. Analytes of interest that can be measured include cellular growth substrates such as carbohydrates, in particular glucose, and molecules that are secreted by cells, such as organic acids (e.g. lactic acid) and peptides. Gaseous analytes include O₂ and CO₂.

Other embodiments of measuring cellular activity using a bioreactor of the invention include measuring cell density, e.g. using optical or non-optical methods. For example, electrical impedance measurements can be performed.

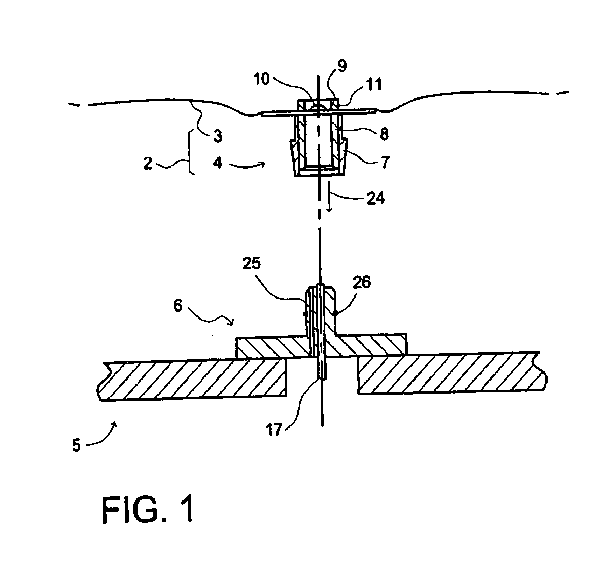

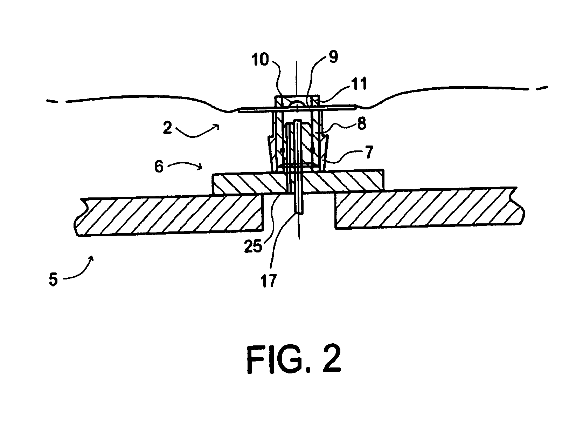

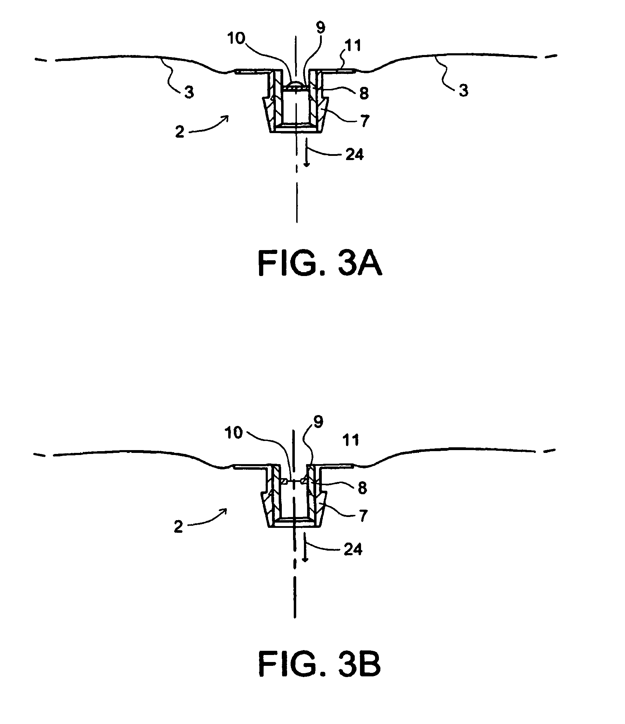

To ensure that the means for monitoring cellular activity, e.g. a sensor element, will not fall dry during cell culturing, for instance during intensive agitation of a bag-shaped bioreactor using the wave motion disclosed in WO2007/00173, the rigid element comprises a wall surrounding said means for monitoring cellular activity, thereby forming a measuring vessel. The dimension and shape of the measuring vessel can vary. Its cross-section may be circular, square or oval. In one embodiment, the vessel is a cylindrical well. Preferably, the diameter of the well is at least 3 times the height of the surrounding wall. In one embodiment, the rigid element comprises a wall protruding into the interior space of the bioreactor and surrounding the sensor element (see Figs. 1 and 2). Thus, a measuring "vessel" is formed which will always contain a required minimum amount of liquid, even if the bioreactor undergoes vigorous rocking. In another embodiment, the surrounding wall faces away from the interior to form a measuring vessel which is located below the surface of the flexible bottom wall when the bioreactor is in use (see Fig. 3). It was found that this positioning of the measuring vessel allows for an optimal exchange between the liquid in the vessel and that of the "bulk liquid" present in the remainder of the bioreactor, such that an even more reliable (on-line) measurement can be obtained.

W02005/068059 relates to a bioprocessing vessel intended for receiving a fluid. The vessel may have a rigid receiver facing an interior and a translucent tube which at its end can be provided with a sensing element. It does not describe a flexible bioreactor wherein measuring means are present in the flexible bottom wall and wherein the measuring means are surrounded by a wall forming a measuring vessel.

It will be appreciated by the person skilled in the art that the rigid element, with measuring means, e.g. a sensor element, is advantageously used in any type of flexible bioreactor that would benefit from being easily secured to a bioreactor support structure in a detachable manner and/or from accurate and reliable monitoring of cellular activity. Preferably, it is a disposable bioreactor. It can be a disposable, sterile tank liner for culturing cells in a liquid medium using in a stirred-tank reactor system. In a preferred embodiment, it is a disposable, sterilisable plastic bag intended for culturing cells in a liquid medium, preferably using wave motion. Of course, the bioreactor is provided with the other necessary elements, for instance means which facilitate the addition or removal of a substance, e.g. liquid medium or a sample. Other accessories and features known in the art may be present as well, including monitoring or sensoring devices other than those described herein above

The invention therefore also discloses a method for monitoring the cellular, e.g. metabolic, activity in a liquid cell culture, wherein a flexible bioreactor provided with a rigid element comprising measuring means, such as transparent bottom section carrying a sensor element, that acts in concert with a probe or other type of detector mounted on a bioreactor support structure. The bioreactor may contain any type of cells that can be cultured in a liquid medium, e.g. prokaryotic or eukaryotic cells, such as algae cells, bacterial cells, mammalian cells, viral cells, or any combination thereof. Whereas the invention is particularly suitable for use in cell culturing, a bioreactor provided with the measuring means as disclosed herein can also be used in other types of applications, for instance in the (on-line) testing or monitoring the properties (e.g. pH or conductivity) of a liquid contained therein such as culture medium without cells or a buffer.

One specific aspect of the invention relates to a bioreactor bag wherein the flexible wall forming the bottom of the bag comprises at least two, preferably three, more preferably four such rigid elements, and wherein at least one of the at least two rigid elements is a sensoring rigid element. Preferably, the multiple rigid elements are substantially arranged along an axis of symmetry of the bottom wall of the bag. The bag can have any shape but preferably has standardised dimensions, for instance it is of an essentially rectangular shape when viewed from the top in an empty state. In one embodiment, it is of rectangular shape and comprising four rigid elements that have a circular cross section. The rigid elements are positioned along the central axis of symmetry parallel to the long edge of the bioreactor bag. Two of the rigid elements are each provided with a distinct sensor element, e.g. an oxygen sensor and a pH sensor. When in use, all four rigid elements contribute to secure and accurate positioning of the bag on the support structure. Additionally, the two sensoring rigid elements allow for on-line monitoring of the metabolic activity of the cells contained in the bag.

It may be advantageous to place the multiple rigid elements, as well as their corresponding adaptor elements on the support structure, in an asymmetrical configuration relative to each other to ensure that the bioreactor can only be detachably secured to a support structure in a single orientation. This is of particular relevance if two or more distinct sensoring rigid elements are involved, each requiring a specific optical probe to read the sensor signal.

In still a further aspect of the invention, a bioreactor bag is provided wherein the flexible wall forming the bottom of the bag is, in addition to at least one rigid insert comprising a measuring vessel, furthermore provided with modulating means for modulating the effective volume of the bag when in use. Said modulating means are capable of acting in co-operation with a pressing device to compartmentalize the bag, thereby allowing for modulating or adjusting the effective volume of the bioreactor during cell culturing. The term "effective volume" is meant to indicate the volume of the bioreactor which can be occupied by the liquid cell culture. Modulating or restraining the effective volume involves in particular the restraining of one or more flexible walls of the bioreactor at the onset of the culturing process, while gradually or stepwise releasing the restrain along with expansion of the cell culture volume.

In one embodiment, the bioreactor is a bag of a substantially rectangular or square shape, and wherein said modulating means comprises at least one rod which is contained in a hollow seam provided at the bottom wall of the bag. To allow for modulation of the effective volume of the bag, the at least one rod is positioned within the perimeter of the bag and arranged parallel to each of a pair of two opposite parallel edges of the bag. Preferably, each rod and its corresponding seam is positioned at a distance from such opposite edge of about one fifth to one third of the distance between the two opposite parallel edges. Preferably, said modulating means comprises two respective such rods contained in two respective such seams, each rod flanking one or more centrally positioned rigid element(s). The at least one rod, together with the bottom and upper flexible wall, is clamped by a clamping device in case the effective volume must be small. For instance, a disposable bag is sold in the "minimal volume" configuration wherein the rods are provided with a clamp, to form a liquid tight barrier between a central part of the bag, e.g. the part comprising the inlet(s), outlet(s) and one or more (sensoring) rigid elements, and the remainder of the bag. The bag can be filled inflated and filled with a small amount of culture medium to start a cell culture at a suitable cell density. Upon an increase in cell density, the clamps can be removed to release the rod and the upper flexible wall, such that the complete volume of the bag becomes available and more growth medium can be added. The clamp is for instance a semi-flexible plastic profile having an omega-shaped cross-section designed such that it can fit tightly yet reversibly around the rod having circular cross-section.

A further aspect of the invention relates to an apparatus for cultivating cells in a flexible bioreactor according to the invention, wherein the apparatus comprises the support structure for receiving or retaining the bioreactor and at least one monitoring device adapted to act in cooperation with the measuring means of the bioreactor to provide an accurate reading of cellular activity in the bioreactor when in use. The monitoring device is for instance an optical fiber probe capable of reading a sensor signal produced by the sensor element contained in the sensoring rigid element. Suitable probes are known in the art, for example it is a polymer optical fiber (POF) supplied by PreSens Precision Sensing GmbH, Regensburg; Germany. In another embodiment, the apparatus is provided with a sensor chamber that allows for the detection of at least one analyte that has passed the membrane. See for example EP1870033. In a preferred embodiment, the apparatus further comprises an adaptor element for cooperation with the rigid element of the bottom wall of the bioreactor such that the rigid element and the adaptor element are securable to one another using vacuum pressure, the adaptor element being arranged for being fixedly connected to the support structure.

The apparatus can be a stirred-tank reactor system capable of holding a disposable flexible tank-liner according to the invention.

In a preferred embodiment, the apparatus is designed for cultivating cells utilizing wave motion using a bag-shaped flexible bioreactor of the invention. It is for instance a rocking apparatus based on the teaching of US 6,190,913, but additionally being provided with adaptor means to accurately secure a culture bag in a predetermined position in the rocking platform. Preferably, the support structure is furthermore provided with at least one optic fiber probe to monitor the metabolic activity of the cells using at least one sensoring rigid element contained in the bottom of the bag. In a specific embodiment, the support structure is provided with at least a first probe for detecting, in cooperation with the respective sensor elements, the pH of a culture, and a second probe for detecting the oxygen level of a culture.

In a preferred embodiment, the apparatus is an apparatus for culturing cells utilizing wave motion of the liquid medium, for example wherein a bioreactor is moved such that it swivels with respect to a substantially horizontal pivot axis while following a cyclical closed-loop path to induce a wave motion in the liquid medium. This type of swivelling is described in detail in WO2007/00173. The apparatus may comprise a driving mechanism for moving at least a part of the support structure for moving the bioreactor to thereby induce the wave motion of the liquid medium. The driving mechanism may preferably comprise at least one cylinder-piston assembly. The cylinder-piston assembly may be a pneumatic or hydraulic one, a pneumatic one being more compatible with large and/or industrial scale cell culturing environments.

It is noted that the intense swivelling motion at high frequencies provided by an apparatus causes a considerable overall impact on such cylinder-piston assemblies. Therefore, to increase the life-time of the cylinder-piston assemblies, the apparatus is advantageously provided with at least one energy absorbing arrangement, preferably a spring-based shock-absorber arranged for abutting the support structure in operation.

The invention is embodied in the independent claims. Further particular embodiments of the invention are set forth in the dependent claims.

Further features, effects and details of embodiments of the invention are described and elucidated below with reference to the schematical figures in the accompanying drawing.
Fig. 1 shows in cross-section a part of a bottom wall of an example of an embodiment of a flexible bioreactor according to the invention.
Fig. 2 shows the example of Fig. 1 again, however, wherein the part of the bottom wall is secured to an adaptor element, the adaptor element being fixedly connected to a support structure for the bioreactor.
Fig. 3 shows in cross-section a part of a bottom wall of an example of further embodiments of a flexible bioreactor according to the invention.
Fig. 4 is a perspective and partial view of an example of a support structure of an example of an embodiment of an apparatus according to the invention.
Fig. 5 shows the example of Fig. 4 again, however together with some additional parts of the support structure.
Fig. 6 is a perspective view of an example of a pressing device used for a flexible bioreactor according to the invention.
Fig. 7 is a bottom view of an example of an embodiment of a flexible bioreactor according to the invention.
Fig. 8 is a cross-section of the bioreactor of Fig. 7 along the line I-I in Fig. 7, wherein the bag is in a condition of having a first effective volume.
Fig. 9 also is a cross-section of the bioreactor of Fig. 7 along the line I-I in Fig. 7, however, wherein the bag is in a condition of having a second effective volume being larger than the first effective volume.

Figures 1, 2, 7, 8 and 9 show a flexible bioreactor 1 for culturing cells in a fluid comprising a liquid medium 20. The bioreactor 1 comprises a flexible wall forming a fluid impermeable bottom wall 2 of the bioreactor when in use. Flexible parts 3 of said flexible bottom wall 2 are interrupted by at least one rigid element 4 of said flexible bottom wall 2. The rigid element 4 allows for detachably securing the bottom wall of the bioreactor to a support structure 5 for receiving or retaining the bioreactor 1.

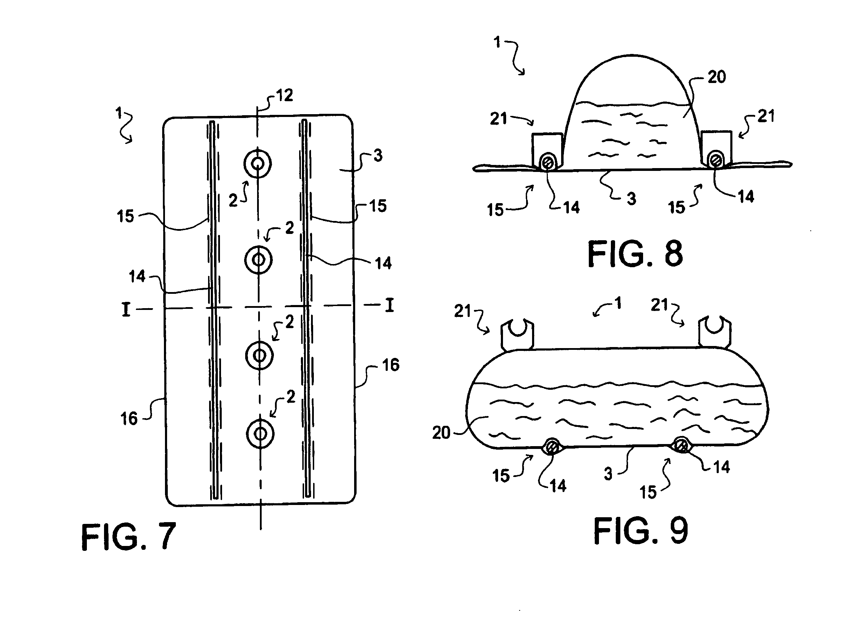

In the example of Fig. 7, the bioreactor 1 has four such rigid elements 2. However, a bioreactor according to the invention may have any suitable number of such rigid elements. In the example of Figs. 1, 2 and 3 a cross section of a part of the bottom wall 2 is shown, which part has one such rigid element 2.

Figs. 1, 2 and 3 show that the rigid element 4 and the flexible parts 3 of the bottom wall 2 are sealingly connected to one another to form a fluid tight connection between one another. In the shown example this is realized in that the rigid element 4 comprises a coupling element 7 that is sealingly connected to the flexible parts 3 of the bottom wall 2 to form a fluid tight connection therewith, and in that the rigid element further comprises an insert element 8 that is sealingly connected to the coupling element 7 to form a fluid tight connection therewith. In the shown example, the coupling element is a hose barb 7 holding the insert element 8 by clamping.

In the lowermost part of Fig. 1, there is shown an adaptor element 6 which is fixedly connected to the support structure 5, for instance by means of bolts or otherwise. The rigid element 4 is arranged for cooperation with the adaptor element 6 such that the rigid element and the adaptor element are securable to one another using vacuum pressure. In order to thus secure the bioreactor 1 to the support structure 5 via the adaptor element 6, bioreactor 1 can be moved in the mounting direction 24. Fig. 2 shows the situation in which the bioreactor is thus secured to the support structure 5. Reference numeral 25 indicates a channel in the adaptor element 6 via which channel the said vacuum pressure can be applied. Reference numeral 26 denotes an O-ring embedded in the periphery of the adaptor element 6.

In the shown example, the insert element 8 of the rigid element 4 has a planar surface 9 of a transparent, non-fluorescent material. The surface 9 is provided with a sensor element 10 facing the interior of the bioreactor 1 to allow for non-invasive monitoring of cellular activity in the bioreactor. In the shown example, the insert element 8 of the rigid element 4 further comprises a wall 11 protruding into the interior space of the bioreactor 1 and surrounding the sensor element 10, thereby forming a measuring vessel. Alternatively, instead of being a part of the insert element 8, such wall 11 can also be a part of the hose barb 7.

Fig. 3A shows an alternative embodiment wherein the wall 11 surrounding the sensor element 10 faces away from the interior space of the bioreactor, to form a measuring vessel that is located below the flexible parts 3 of the bioreactor when in use.

Fig. 3B corresponds to Fig. 3A with the exception that the rigid element is provided with a membrane 110.

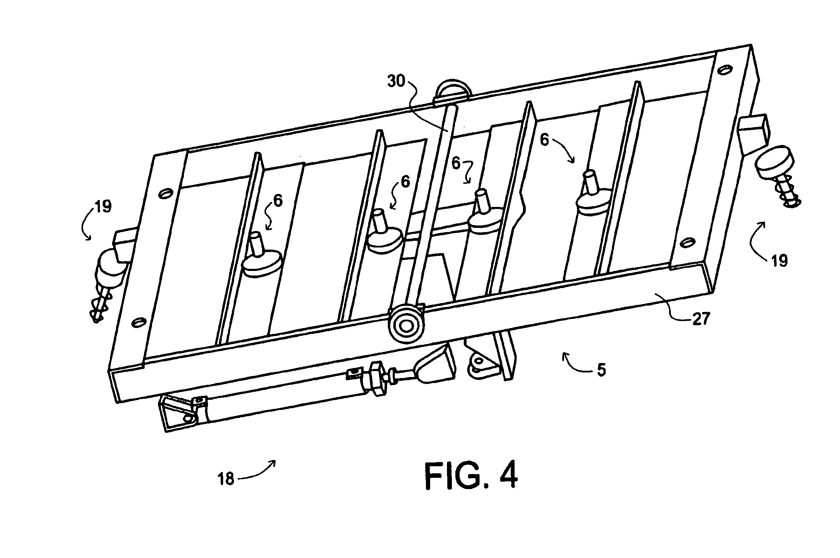

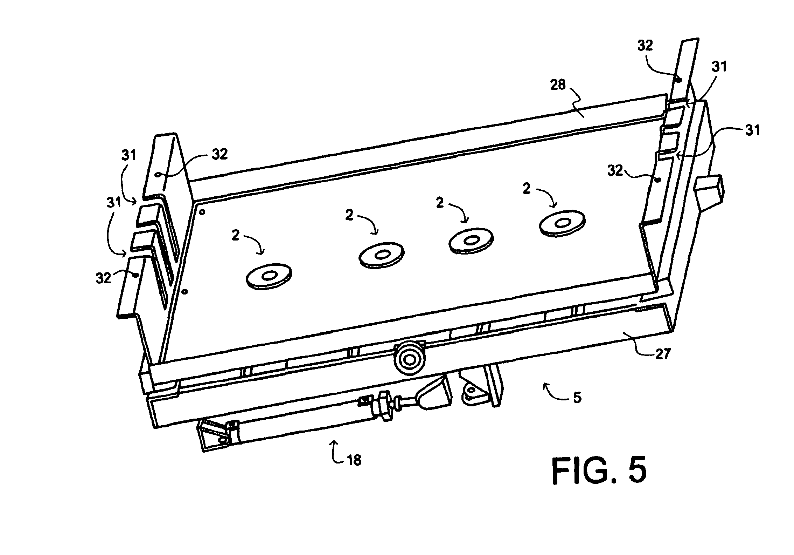

Although, as described above, a bioreactor of the invention can for example be a disposable, sterile tank liner for culturing cells in a stirred-tank reactor system, it is hereinafter assumed that the bioreactor 1 is a disposable, sterilisable plastic bag 1 for culturing cells utilizing wave motion. Such cell culturing is done in an apparatus according to the invention, which apparatus comprises the support structure 5 for receiving or retaining the bioreactor 1. Figs. 4 and 5 show some further details of a support structure of such an apparatus.

Fig. 4 shows a first frame structure 27 of the support structure 5. The adaptor elements 6, in this case four, are provided on this first frame structure 27. Reference numeral 30 denotes a swivelling axis of the support structure 5. The apparatus comprises a driving mechanism for moving at least a part of the support structure 5 for moving the bioreactor 1 to thereby induce wave motion of the liquid medium 20. For simplicity, this driving mechanism is not shown in detail. In fact, there is only shown just one cylinder-piston assembly 18 being part of such driving mechanism. The apparatus is further provided with at least one energy absorbing arrangement as described earlier. This is shown in Fig. 4 by means of two spring-based shock-absorbers 19 arranged for abutting the first frame structure 27 of the support structure 5 in operation.

Fig. 5 shows a second frame structure 28 fitted on top of the first frame structure 27. The second frame structure 28 has a more or less tray-like shape with upstanding side-walls for receiving the bioreactor bag 1 shown in Figs. 7, 8 and 9. For simplicity, the bioreactor 1 is not fully shown in Fig. 5, only the four rigid elements 2 of the bioreactor bag 1 are shown in Fig. 5.

Reference is now made to Figs. 7, 8 and 9, which figures show the bioreactor bag 1. In the shown example, the flexible wall 2 forming the bottom of the bag 1 comprises four rigid elements 4 substantially arranged along an axis 12 of symmetry of the bottom wall 2 of the bag 1. For example, the two middle ones of the shown four rigid elements 4 are sensoring rigid elements, while the two outer ones are securing rigid elements. For each of the sensoring rigid elements the apparatus comprises, as explained, an optical fiber 17 connected to the respective adaptor element 6 (see Figs. 1 and 2), said optical fiber being arranged such that it can read a sensor signal generated by the sensor element 10 of the sensoring rigid element 4.

In the shown example, the multiple rigid elements 4, as well as their corresponding adaptor elements 6 are placed on the support structure 5 in an asymmetrical configuration relative to each other to ensure, as explained before, that the bioreactor can only be detachably secured to a support structure in a single orientation. This is realized in that in Fig. 5 each pair of adjacent ones of the rightmost three rigid elements 2 have a first, equal distance between them, while the leftmost two adjacent rigid elements 2 have a second distance between them, said second distance differing from said first distance. The analogous observation applies regarding the lowermost three rigid elements 2 versus the uppermost two in Fig 7, as well as of course regarding the distances between the adaptor elements 6 of Fig. 4.

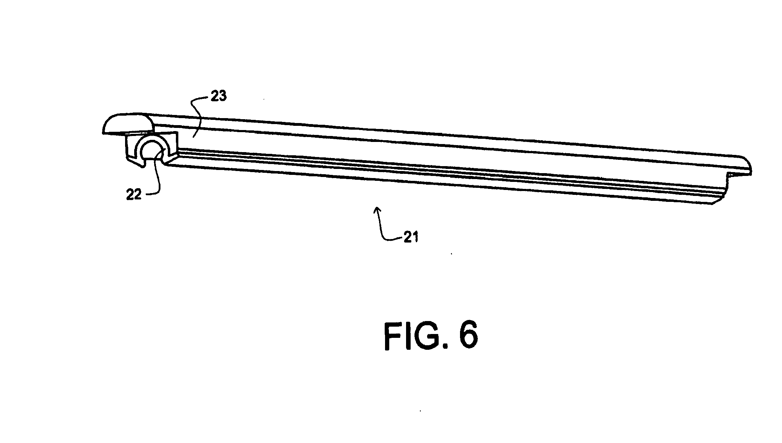

In Figs. 7, 8 and 9, the flexible wall 2 forming the bottom of the bag 1 is furthermore provided with modulating means 14, 15 for modulating the effective volume of the bag when in use, said modulating means being capable of acting in co-operation with a pressing device 21 shown in Fig. 6. This is elucidated as follows.

The shown bioreactor bag 1 is of a substantially rectangular shape. Said modulating means comprises two rods 14, each contained in a respective hollow seam 15 provided at the bottom wall 2 of the bag. The rods 14 are arranged parallel to one another and parallel to each of a pair of two opposite parallel edges 16 of the bag. For each of the two rods 14, there is provided a corresponding pressing device 21. The pressing device 21 is a semi-flexible plastic profile 22 having an omega-shaped cross-section designed such that it can fit tightly yet reversibly around the rod 14 having circular cross-section. The omega-profile is embedded in a body profile 23 of the pressing device 21.

Fig. 8 shows a condition of the bag 1, wherein such tight fit is present, the bag thus being in a first condition of having a first effective volume. In this first condition, the two opposite ends of the body profile 23 are to be received into slits 31 in the upstanding walls of the second frame structure 28 (see Fig. 5).

Fig. 9 shows a condition of the bag 1, wherein such tight fit has been cancelled, the bag thus being in a second condition of having a second effective volume being larger than the first effective volume. Optionally, the pressing device 21 can be designed such that in this second condition the two opposite ends of the body profile 23 can be fixedly connected to the second frame structure 28 via screw receiving openings 32 in top flanges of the upstanding walls of the second frame structure 28 (see Fig. 5). In this second condition the pressing devices 21 help to keep the bag 1 in place during (swivelling) operation of the apparatus. Preferably, in this second condition, the pressing devices 21 are mounted upside-down relative to the orientation of Fig. 8, while in this second condition the areas of the pressing devices 21 which contact the bag 1 have been designed with the aim of being in non-slippery and nondestructive contacting engagement with the bag 1.

## Claims

1. A flexible bioreactor intended for culturing cells in a liquid medium (20), the bioreactor (1) comprising a flexible wall forming a fluid impermeable bottom wall (2) of the bioreactor when in use, wherein flexible parts (3) of said flexible bottom wall are interrupted by at least one rigid element (4) of said flexible bottom wall, the rigid element (4) and the flexible parts (3) being connected fluid tightly, the rigid element comprising measuring means (10; 110) allowing for non-invasive monitoring of cellular activity in said bioreactor when in use, wherein the rigid element (4) comprises a wall (11) surrounding said at least one measuring means, thereby forming a measuring vessel.

2. Bioreactor according to claim 1, wherein the rigid element (4) comprises a coupling element (7) that is sealingly connected to the flexible parts (3) of the bottom wall (2) to form a fluid tight connection therewith, and wherein the rigid element further comprises an insert element (8) that is sealingly connected to the coupling element to form a fluid tight connection therewith.

3. Bioreactor according to claim 2, wherein the coupling element is a hose barb (7) holding the insert element (8) by clamping, the insert element preferably being wedge-shaped.

4. Bioreactor according to any one of claims 1 to 3, wherein the measuring means are formed by the rigid element (4) having a surface (9), preferably a planar surface, of a transparent, non-fluorescent material, said surface being provided with at least one sensor element (10) facing the interior of the bioreactor (1).

5. Bioreactor according to claim 4, wherein the at least sensor element (10) is a sensor containing a luminescent or fluorescent dye or a sterilizable electrode.

6. Bioreactor according to any one of claims 1 to 5, wherein the measuring means are formed by the rigid element (4) being interrupted by a membrane (110) allowing the passage of at least one analyte, the analyte being an indicator of cellular activity, preferably wherein the membrane (110) is a dialysis membrane, more preferably a cellulose acetate membrane.

7. Bioreactor according to any one of claims 1 to 6, wherein wall (11) faces away from the interior space of the bioreactor (1) to form a measuring vessel which, when the bioreactor is in use, is located below the flexible parts (3) of the bottom wall (2).

8. Bioreactor according to any one of the above claims, wherein the rigid element furthermore allows for detachably securing the bottom wall of the bioreactor to a support structure (5) for receiving or retaining the bioreactor.

9. Bioreactor according to any one of the above claims, wherein the flexible parts (3) of the bottom wall (2) are made of a transparent or translucent flexible plastic, preferably polyethylene or polypropylene.

10. Bioreactor according to any one of the above claims, wherein the rigid element (4) is at least partly made of a transparent, hardened plastic, preferably polyethylene or polycarbonate.

11. Bioreactor according to any one of claims 1-10, being a disposable, sterilisable plastic bag (1), preferably a bag for culturing cells utilizing wave motion.

12. Bioreactor according to any of the preceding claims, wherein the flexible wall (2) forming the bottom of the bag is furthermore provided with modulating means (14, 15) for modulating the effective volume of the bag (1) when in use, said modulating means being capable of acting in co-operation with a pressing device (21).

13. An apparatus for cultivating cells in a flexible bioreactor (1) according to any one of claims 1 to 12, the apparatus comprising the support structure (5) for receiving or retaining the bioreactor and at least one monitoring device (17) adapted to act in cooperation with the measuring means (10; 110) of the bioreactor to allow for non-invasive monitoring of cellular activity in the bioreactor when in use.

14. Apparatus according to claim 13, wherein said monitoring device is an optical fiber (17), said optical fiber being arranged such that it can read a sensor signal generated by a sensor element (10) provided at the bottom wall (2) of the bioreactor (1).

15. Apparatus according to claim 14, wherein the optical fiber (17) is connected to the adaptor element (6).

## Patentansprüche

1. Flexibler Bioreaktor zur Kultivierung von Zellen in einem Flüssigmedium (20), wobei der Bioreaktor (1) eine flexible Wand umfasst, die bei Verwendung eine flüssigkeitsundurchlässige untere Wand (2) des Bioreaktors bildet, wobei flexible Teile (3) der flexiblen unteren Wand durch mindestens ein starres Element (4) der flexiblen unteren Wand unterbrochen sind, wobei das starre Element (4) und die flexiblen Teile (3) flüssigkeitsdicht verbunden sind und das starre Element Messmittel (10;110) umfasst, die eine nichtinvasive Überwachung der Zellaktivität in dem Bioreaktor, wenn dieser verwendet wird, ermöglichen, wobei das starre Element (4) eine Wand (11) umfasst, die mindestens ein Messmittel umgibt und so einen Messbehälter bildet.

2. Bioreaktor nach Anspruch 1, wobei das starre Element (4) ein Kopplungselement (7) umfasst, das abdichtend mit den flexiblen Teilen (3) der unteren Wand (2) verbunden ist, um damit eine flüssigkeitsdichte Verbindung zu bilden, und wobei das starre Element ferner ein Einsatzelement (8) umfasst, das abdichtend mit dem Kopplungselement verbunden ist, um damit eine flüssigkeitsdichte Verbindung zu bilden.

3. Bioreaktor nach Anspruch 2, wobei das Kopplungselement ein Schlauchhaken (7) ist, der das Einsatzelement (8) durch Klemmen hält, wobei das Einsatzelement vorzugsweise keilförmig ist.

4. Bioreaktor nach einem der Ansprüche 1 bis 3, wobei die Messmittel durch das starre Element (4) mit einer Oberfläche (9) gebildet werden, vorzugsweise einer ebenen Oberfläche, aus einem transparenten, nicht fluoreszierenden Material, wobei die Oberfläche mit mindestens einem zum Innenraum des Bioreaktors (1) gerichteten Sensorelement (10) versehen ist.

5. Bioreaktor nach Anspruch 4, wobei das mindestens eine Sensorelement (10) ein Sensor ist, der einen lumineszierenden oder fluoreszierenden Farbstoff oder eine sterilisierbare Elektrode enthält.

6. Bioreaktor nach einem der Ansprüche 1 bis 5, wobei die Messmittel durch das starre Element (4) gebildet werden, das von einer Membran (110) unterbrochen wird, um den Durchgang von mindestens einem Analyten, der ein Anzeiger von Zellaktivität ist, zu ermöglichen, wobei die Membran (110) vorzugsweise eine Dialysemembran, noch bevorzugter eine Cellulose-Acetat-Membran ist.

7. Bioreaktor nach einem der Ansprüche 1-6, wobei die Wand (11) vom Innenraum des Bioreaktors (1) weg gerichtet ist, um einen Messbehälter zu bilden, der bei Verwendung des Bioreaktors unter den flexiblen Teilen (3) der unteren Wand (2) angeordnet ist.

8. Bioreaktor nach einem der vorhergehenden Ansprüche, wobei das starre Element ferner das lösbare Befestigen der unteren Wand des Bioreaktors an einer Stützstruktur (5) zum Aufnehmen oder Halten des Bioreaktors ermöglicht.

9. Bioreaktor nach einem der vorhergehenden Ansprüche, wobei die flexiblen Teile (3) der unteren Wand (2) aus einem durchsichtigen oder durchscheinenden flexiblen Kunststoff hergestellt sind, vorzugsweise Polyethylen oder Polypropylen.

10. Bioreaktor nach einem der vorhergehenden Ansprüche, wobei das starre Element (4) mindestens teilweise aus einem durchsichtigen, gehärteten Kunststoff hergestellt ist, vorzugsweise Polyethylen oder Polycarbonat.

11. Bioreaktor nach einem der Ansprüche 1-10, der ein sterilisierbarer Einweg-Kunststoffbeutel (1) ist, vorzugsweise einen Beutel zum Kultivieren von Zellen unter Verwendung einer Wellenbewegung.

12. Bioreaktor nach einem der vorhergehenden Ansprüche, wobei die flexible Wand (2), die den Boden des Beutels bildet, ferner mit Anpassungsmitteln (14, 15) zum Anpassen des effektiven Volumens des Beutels (1) versehen ist, wobei die Anpassungsmittel in der Lage sind, mit einer Pressvorrichtung (21) zusammenzuarbeiten.

13. Vorrichtung zur Kultivierung von Zellen in einem flexiblen Bioreaktor (1) nach einem der Ansprüche 1 bis 12, umfassend die Stützstruktur (5) zum Aufnehmen oder Halten des Bioreaktors und mindestens eine Überwachungsvorrichtung (17), die geeignet ist, mit den Messmitteln (10; 110) des Bioreaktors zusammenzuarbeiten, um bei Verwendung des Bioreaktors eine nichtinvasive Überwachung der Zellaktivität in dem Bioreaktor zu ermöglichen.

14. Vorrichtung nach Anspruch 13, wobei die Überwachungsvorrichtung eine optische Faser (17) ist, die so angeordnet ist, dass sie ein Sensorsignal, erzeugt von einem an der unteren Wand (2) des Bioreaktors (1) bereitgestellten Sensorelement (10), lesen kann.

15. Vorrichtung nach Anspruch 14, wobei die optische Faser (17) mit dem Adapterelement (6) verbunden ist.

## Revendications

1. Bioréacteur flexible destiné à la culture de cellules dans un milieu liquide (20), le bioréacteur (1) comprenant une paroi flexible formant une paroi inférieure imperméable aux fluides (2) du bioréacteur quand il est en utilisation, où les parties flexibles (3) de ladite paroi inférieure flexible sont interrompues par au moins un élément rigide (4) de ladite paroi inférieure flexible, l'élément rigide (4) et les parties flexibles (3) étant reliés de manière étanche aux fluides, l'élément rigide comprenant des moyens de mesure (10 ; 110) permettant le suivi non invasif de l'activité cellulaire dans ledit bioréacteur quand il est en utilisation, où l'élément rigide (4) comprend une paroi (11) entourant ledit au moins un moyen de mesure, en formant ainsi un récipient de mesure.

2. Bioréacteur selon la revendication 1, où l'élément rigide (4) comprend un élément de couplage (7) qui est relié de manière étanche aux parties flexibles (3) de la paroi inférieure (2) pour former une liaison étanche aux fluides avec celles-ci, et où l'élément rigide comprend en outre un élément d'insertion (8) qui est relié de manière étanche à l'élément de couplage pour former avec lui une liaison étanche aux fluides.

3. Bioréacteur selon la revendication 2, où l'élément de couplage est une barbe de tuyau (7) maintenant l'élément d'insertion (8) par serrage, l'élément d'insertion étant de préférence en forme de coin.

4. Bioréacteur selon l'une quelconque des revendications 1 à 3, où les moyens de mesure sont formés par l'élément rigide (4) ayant une surface (9), de préférence une surface plane, d'un matériau non fluorescent transparent, ladite surface étant munie d'au moins un élément capteur (10) tourné vers l'intérieur du bioréacteur (1).

5. Bioréacteur selon la revendication 4, où le au moins un élément capteur (10) est un capteur contenant un colorant luminescent ou fluorescent ou une électrode stérilisable.

6. Bioréacteur selon l'une quelconque des revendications 1 à 5, où les moyens de mesure sont formés par l'élément rigide (4) étant interrompu par une membrane (110) permettant le passage d'au moins un analyte, l'analyte étant un indicateur d'activité cellulaire, de préférence où la membrane (110) est une membrane de dialyse, de préférence encore une membrane en acétate de cellulose.

7. Bioréacteur selon l'une quelconque des revendications 1 à 6, où la paroi (11) est tournée à distance de l'espace intérieur du bioréacteur (1) pour former un récipient de mesure qui, quand le bioréacteur est en utilisation, est situé en dessous des parties flexibles (3) de la paroi inférieure (2).

8. Bioréacteur selon l'une quelconque des revendications précédentes, où l'élément rigide permet en outre la fixation amovible de la paroi inférieure du bioréacteur à une structure de support (5) pour recevoir ou retenir le bioréacteur.

9. Bioréacteur selon l'une quelconque des revendications précédentes, où les parties flexibles (3) de la paroi inférieure (2) sont faites d'un plastique flexible transparent ou translucide, de préférence le polyéthylène ou le polypropylène.

10. Bioréacteur selon l'une quelconque des revendications précédentes, où l'élément rigide (4) est fait au moins en partie d'un plastique durci transparent, de préférence le polyéthylène ou le polycarbonate.

11. Bioréacteur selon l'une quelconque des revendications 1-10 qui est un sac plastique stérilisable jetable (1), de préférence un sac pour la culture de cellules utilisant un mouvement ondulatoire.

12. Bioréacteur selon l'une quelconque des revendications précédentes, où la paroi flexible (2) formant le fond du sac est munie en outre de moyens modulateurs (14, 15) pour moduler le volume effectif du sac (1) quand il est en utilisation, lesdits moyens modulateurs étant capables d'agir en coopération avec un dispositif presseur (21).

13. Appareil pour cultiver des cellules dans un bioréacteur flexible (1) selon l'une quelconque des revendications 1 à 12, l'appareil comprenant la structure de support (5) pour recevoir ou retenir le bioréacteur et au moins un dispositif de suivi (17) adapté pour agir en coopération avec les moyens de mesure (10 ; 110) du bioréacteur pour permettre le suivi non invasif de l'activité cellulaire dans le bioréacteur quand il est en utilisation.

14. Appareil selon la revendication 13, où ledit dispositif de suivi est une fibre optique (17), ladite fibre optique étant disposée de telle façon qu'elle peut lire un signal de capteur généré par un élément capteur (10) prévu au niveau de la paroi inférieure (2) du bioréacteur (1).

15. Appareil selon la revendication 14, où la fibre optique (17) est reliée à l'élément adaptateur (6).
